# EUROPEAN PATENT APPLICATION

(11) **EP 4 725 443 A1**
(43) Date of publication of application: **15.04.2026**
(21) Application number: 24206309.7
(22) Date of filing: 14.10.2024
(51) Int. Cl.: A61B 34/37, A61B 90/25, A61B 90/00, A61B 90/50, A61B 34/20

(54) **A SURGICAL ROBOTIC SYSTEM COMPRISING A SURGICAL MICROSCOPE**

(71) Applicant: Microsure B.V., 5692 EA Son (NL)
(72) Inventor: DIJKKAMP, Dirk, 5581 TK Waalre (NL); BORGGREVE, Herman, 5582 KG Waalre (NL); KONERT, Tom, 1103 BJ Amsterdam (NL)
(74) Representative: DTS Patent- und Rechtsanwälte PartmbB

(57) **Abstract**

The present invention provides for a surgical robotic system (100) for performing a microsurgery or supermicrosurgery. The surgical robotic system comprises a control system (102) and a robotic system (104). The robotic system (104) comprises a monitoring unit (106) for monitoring a surgical area, and at least one manipulator arm (108) for manipulating a surgical instrument, wherein the control system (102) is configured to control the monitoring unit (106) and the at least one manipulator arm (108), and wherein the monitoring unit (106) and the at least one manipulator arm (108) are configured and designed to have a common reference frame.

## Description

The present invention relates to the field of surgical robotics and, more specifically, to systems and methods for performing microsurgery or supermicrosurgery. In particular, a surgical robotic system is provided that incorporates a microsurgical robot and a surgical microscope as an integral part of one single unit to enhance surgical precision and control mechanism in microsurgical procedures.

Supermicrosurgery involves intricate surgical procedures performed at a microscopic level, requiring exceptional precision, stability and visual acuity. The surgical robotic systems include robotic manipulators that are designed specifically for microsurgical tasks and for performing precise surgical handlings on the sub-millimetre scale. The microsurgical robots can help stabilize the surgeon's hand movements, reducing the effects of tremors and allowing for more precise manipulation of delicate tissues and structures. The robotic manipulators thus can facilitate accurate movements with micrometre precision without substantial tremor, thereby superseding the limitations of the human hand. However, visual perception still remains crucial for surgeons to effectively navigate and manipulate the robotic manipulators during the surgical procedures.

Currently, the robotic manipulators are used in combination with an external (independent) digital (stereo) surgical microscope to enable the surgeon to visualize the operating procedures with sufficient resolution. To this end, the surgical systems typically include a microscope that is mounted on a separate cart. The positioning of the microscope is carried out in a semi-manual way involving interaction of an operator.

The provision of an additional unit for the microscope (e.g. a microscope cart) has several disadvantages. For example, in operating theatres with a limited space, a separate cart takes up a further space near an operating table.

Further, the microscopic unit includes various components (e.g. a camera head and/or a suspension column) that are typically rigid and bulky. An optimal positioning and arrangement of the microscope unit with respect to the microsurgical manipulators can be rather problematic. This may even lead to a suboptimal positioning due to volume constraints when used together with a robotic platform, and to a higher risk of collisions.

One further drawback of the provision of a detached and independent microscope unit resides in incompatible coordinate systems (or incompatible measurement scales) of the microscope unit (i.e. its camera or sensors) and the manipulator unit.

Not having a common reference for the microsurgical robotic manipulator and the microscope unit restricts a seamless and detailed view for a surgeon when conducting delicate surgical maneuvers. Further, lack of a common reference lead to an increase of the complexity in the data processing and analysis. Additional steps may be required to transform or align the microscope unit. This, in turn, may cause delays in the surgical procedures and potentially introduces inaccuracies.

In other words, without a shared frame of reference for the robotic manipulator and the microscope unit the flexibility and accuracy of microsurgical procedures (or measurements) may be compromised, leading to suboptimal results in applications where precision is critical.

It is therefore an object of the present invention to address the aforesaid drawbacks by providing a surgical robotic system including a fully integrated and interoperable microscope unit and robotic microsurgical manipulator to thereby enable the surgeons an improved controllability and accuracy during procedures.

This object is achieved in the present invention by a surgical robotic system for performing a microsurgery or supermicrosurgery according to claim 1. Accordingly, a surgical robotic system is provided that comprises:
a control system and a robotic system, the control system being in communication with the robotic system,
wherein the robotic system comprises
   - a monitoring unit (or a visualization unit) for monitoring a surgical area, and
   - at least one manipulator arm for manipulating a surgical instrument,
wherein the control system is configured to control the monitoring unit and the at least one manipulator arm and
wherein the monitoring unit and the at least one manipulator arm are configured and designed to have a common reference frame.

The invention is based on the basic idea that by physically integrating the monitoring unit (e.g. the microscope unit) and the robotic manipulator arm in one single robotic system and by providing one (central) control system for controlling both the monitoring unit and the at least one manipulator arm, a complete, one-stop shop solution for performing supermicrosurgery procedures with enhanced tractability and controllability is provided. In particular, the functionality and capability of the surgical robotic system is significantly increased due to the (physical) integration of the monitoring unit (e.g. the microscope) and the at least one manipulator arm onto one single support structure (e.g. a housing of the robotic system). In particular, by designing and configuring the surgical robotic system such that the monitoring unit and the at least one manipulator arm provide for a common frame of reference the interoperability between the monitoring unit and the at least one manipulator arm is improved while the complexity of the surgical robotic system is reduced by having one control system. The monitoring unit may be embodied as a microscope unit, comprising a microscope. In particular, the microscope can be a digital microscope. The digital microscope may provide enlarged views on the surgical intervention site in real-time (i.e. shown, presented at the same time as events actually happen, taking into account and including the very short time needed).

For example, by installing (or mounting) the microscope unit (e.g. including a camera unit or a sensor unit) in an appropriate position on the robotic microsurgical manipulator (e.g. by mounting a 3D camera on the frame of the robotic system or on the support frame of the robotic manipulator arm), it is possible to use a standardized (common) coordinate system to represent the spatial orientation, the position of the captured images or videos, and position of the robotic manipulator arm. This configuration further establishes a consistent and uniform frame of reference that allows for detailed real-time visualization and improved accuracy in the surgical maneuvers by the surgeons.

In particular, when the camera and the robotic arms are in the same reference frame, it would also allow easy semi-automatic camera positioning, e.g. automatic tracking of the positions of the tips of the microsurgical instruments, semi-automatic zooming in/out depending on the type of movements of the robotic arms. In addition, the need for repositioning of the microscope unit by a separate operator would vanish, which increases overall efficiency and saves the time. The cost of such integrated and compact surgical robotic system is also significantly decreased compared to current situation with separate units.

One further advantage of the surgical robotic system according to the present invention is freeing up the precious floorspace close to the operating table. The issues with the footprint of the microscope cart will also be resolved. In addition, integrating the microscope unit in the design of the robotic manipulator arm would reduce the risk of collision and improve remote positioning and control of the monitoring unit (e.g. the camera unit) by the surgeon taking into account any volume constraints by design.

Further, the present invention having a common control unit provide for an easier handling (troubleshooting) in cases when issues arise in the microscope unit (e.g. camera units). The lack of a consistent reference and/or accessible control parameters makes the process of the troubleshooting more challenging. In particular, surgeons cannot identify and address, for example, calibration errors, alignment discrepancies, or other technical issues across camera units and/or manipulator unit without one accessible control system during surgical procedures.

In particular, the monitoring unit is installed and arranged in a support frame of the robotic system. Preferably, the monitoring unit is mounted onto a suspension frame. In other words, the at least one manipulator arm and the monitoring unit both are mounted onto (carried by) one support frame, i.e. the suspension frame.

Advantageously, by structurally unifying the monitoring unit and the manipulator onto one single frame, a common reference or coordinate system in the surgical robotic system is established. This enables accurate movements of the manipulator arm, seamless data integration, interoperability, simplified alignment, and more reliable microsurgical procedures involving interaction of multiple units.

In particular, the monitoring unit is mounted onto the suspension frame, for example, via a robotic assembly. Preferably, the robotic assembly comprises a selective compliance articulated robot arm.

By integrating the monitoring unit (e.g. a camera) into the robotic system, the invention enhances surgical precision, improves depth perception, and provides real-time visual feedback for improved microsurgical outcomes.

In particular, the control system comprises a first control unit for controlling movements of the at least one manipulator arm, a second control unit for positioning and controlling the monitoring unit, and an interface unit for communicating with the first and second control units.

In particular, the monitoring unit, via the second control unit or the control system, is configured to track a position of a surgical instrument that is carried by the manipulator arm. In particular, the monitoring unit is further configured to zoom in or zoom out depending on the movements of the manipulator arm.

Advantageously, by establishing a common frame of reference, the camera can provide accurate and reliable spatial information (or a consistent scene reconstruction), when tracking the surgical instrument.

In particular, the at least one manipulator arm comprises a first arm having a first end that is connected to an end effector, and a second end that is connected to a second arm, wherein the end effector is configured to hold the surgical instrument. Advantageously, a reference point within the coordinate system from which all positions are measured (i.e. the origin point) and the orientation of the coordinate axes for establishing the directions for positive and negative values along each axis can be uniformly defined for the manipulator arm and the monitoring unit. This, in turn, further ensures consistency and allows for proper alignment in the surgical system.

In particular, the support frame has a mounting surface, wherein the monitoring unit is mounted onto a center of the mounting surface.

In particular, the first arm is connected to the end effector via a first joint and to the second arm via a second joint.

In particular, the manipulator arm further comprises a third arm that is connected to the second arm via a third joint, preferably the first, second and third joints, each comprising a roll and a pitch.

In particular, the robotic system comprises two manipulator elements, each comprising a first arm, a second arm and a third arm.

In particular, the second control unit or the control system comprises a computing device for controlling the monitoring unit, wherein the computing device is configured to adjust a focus point, an autofocus and/or a zooming range.

In particular, the second control unit or the control system further comprises a controller for controlling movements of the robotic assembly.

Advantageously, the transmission of visual information from the surgical treatment area (via the monitoring unit, e.g. the camera feeds) in concert with the movements of the manipulator arm provides a precise, real-time guidance, and superimposes additional controlling aspects in the surgical field, consistently assisting the surgeon during complex procedures.

In particular, the second control unit of the control system further comprises an image processing unit, a display, a storage unit and a knowledge base.

In particular, the first control unit of the control system comprises a motion control unit for controlling movements of the at least one manipulator arm.

In particular, the monitoring unit comprises a digital imaging sensor and/or a digital 3D camera.

In particular, the monitoring unit has resolutions up to 4K-3D (e.g. Olympus Orbeye, surgical microscope), magnification up to 1:12 (e.g. Optical 1:6 and digital 1:1.5 or 1:2). The image sensor can be 4K and the output formats can be 4K (3D/2D) or HD (3D/2D). The signal output (transmission output) can be 3G-SDI or HD-SDI)

In particular, the interface unit in the control system comprises

It is shown in
- **Fig. 1:**: a block diagram of a surgical robotic system;
- **Fig. 2:**: a block diagram of a surgical robotic system, indicating the components of a control system and a robotic system;
- **Fig. 3:**: a schematic view of the surgical robotic system, indicating the at least one manipulator arm and a monitoring unit;
- **Fig. 4:**: a schematic view of the surgical robotic system, indicating a portion of the robotic system; and
- **Fig. 5:**: a functional block diagram of the surgical robotic system.

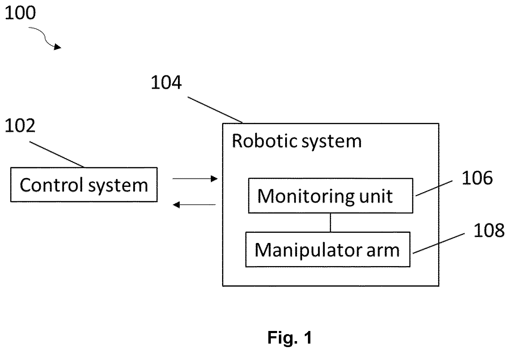

**Fig. 1** illustrates a block diagram of a surgical robotic system 100 for performing a microsurgery or supermicrosurgery. The surgical robotic system 100 comprises a control system 102 and a robotic system 104.

The control system 102 is configured to control the robotic system 104 (e.g. a microsurgical robot).

The robotic system 104 comprises a monitoring unit 106 and at least one manipulator arm 108.The manipulator arm 108 is configured to help the surgeon to perform delicate surgical manoeuvres in a body portion of a patient (i.e. the surgical/treating area).

The manipulator arm 108 includes, for example, robotic arms equipped with microsurgical instruments, such as micro-scissors, micro-forceps, and micro-sutures.

The manipulator arm 108 advantageously allows for precise and stable manipulation of delicate tissues and structures.

The monitoring unit 106 is configured to facilitate imaging and visualization of the surgical area.

For example, the monitoring unit 106 comprises high-resolution cameras (e.g. a digital 3D camera) and/or fluorescence imaging, to provide real-time visualization of the surgical area. This enhances the surgeon's ability to identify and manipulate tiny structures accurately.

In particular, the camera is configured to capture high-resolution images of the surgical site and to provide a three-dimensional view of the surgical area. This in turn provides a clear and detailed view for the surgeon. Further, it enhances depth perception and allows for more accurate assessment of anatomical structures and spatial relationships.

The control system 102 of the surgical robotic system 100 is configured to control both the monitoring unit 106 and the manipulator arm 108.

In particular, in this way a control system 102 is provided to the surgeon that allows him to operate the manipulator arm 108 (e.g. microsurgical robots) as well as the monitoring unit 106 from a single integrated unit. The control system 102 provides a user-friendly interface, real-time visualization of the surgical area, and intuitive control mechanisms to facilitate seamless and precise manipulation of the manipulator arm 108.

For example, the control system 102 is a surgeon interface unit (e.g. Sill-cart or SSU-cart) and the robotic system 104 is a patient interface unit (e.g. Pill-cart or PSU-cart). The surgeon operating the control system 102 is therefore provided with a combined (integrated) control system enabling him to directly control the monitoring unit and the manipulator arm during the microsurgical procedures.

In particular, the surgeon is enabled via the control system 102 to adjust magnification of the monitoring unit 106. For example, surgeons can zoom in or out to focus on specific areas of interest, enabling a closer examination of anatomical structures.

In addition, the monitoring unit 106 (e.g. PSU-microscope) that typically has a wide field of view (providing a broad visual coverage of the surgical areas) allows surgeons to obtain a comprehensive view of the operative area. This, in turn, enhances situational awareness and facilitates an efficient surgical performance via the manipulator arm 108.

In particular, the monitoring unit 106 and the at least one manipulator arm 108 are configured and designed to have a common reference frame.

In other words, the surgical robotic system 100 is designed (and configured) such that a common (standardized) coordinate system is used for the monitoring unit and the at least one manipulator arm 108 for representing the spatial orientation, position of the captured images or videos and position of the manipulator arm within the surgical area.

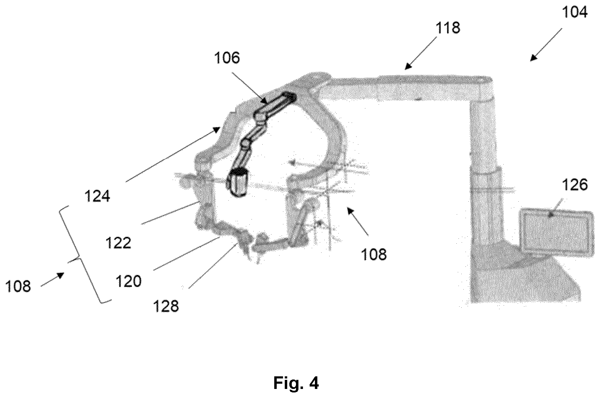

For example, in Fig. 4 the surgical robotic system 100 comprises two manipulator arms 108 that have a common reference frame with the monitoring unit 106.

Advantageously, by way of this configuration the surgical robotic system 100 provides for a consistent and uniform frame of reference that allows for accurate analysis and surgical performance.

In particular, a single integrated control system (e.g. a surgeon interface unit) is provided for surgeons to control vision and motion, thereby optimizing the eye-hand coordination and reducing learning curve.

In addition, by way of the surgical robotic system integrating the imaging capabilities and the robotic manipulations the space in the operating room is better managed. The requirement for draping is reduced, which also affects the amount of waste generated.

Further, the surgical robotic system improves the real-time visualization of the surgical area due to better focus of the monitoring unit. In particular, due to the capability of the surgical system that is configured such a monitoring unit (e.g. the integrated camera) is configured to track the instrument tip carried by the manipulator arm. This configuration further improves the depth of focus.

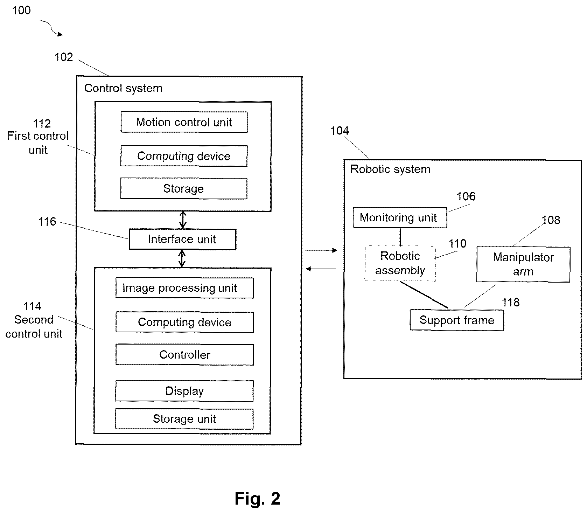

**Fig. 2** shows a block diagram of a surgical robotic system, indicating the components of a control system 102 and the robotic system 104.

Optionally, a robotic assembly 110 may be further included in the surgical robotic system 100 for mounting the monitoring unit 106 onto the support frame 118 (shown as a broken line in Fig. 2).

In particular, the control system 102 comprises a first control unit 112 for controlling movements of the manipulator arm 108.

Further, a second control unit 114 is included in the control system 102 for positioning and controlling the at least one monitoring unit 106.

The control system 102 further comprises an interface unit 116 in a communication with the first and second control units 112, 114.

For example, the first control unit 112 may comprise a motion control unit, a storage and/or a computing device.

Advantageously, the surgical robotic system 100 benefits from ergonomic design. The surgical robotic system takes into account the ergonomic considerations for the surgeon, ensuring optimal positioning and comfort during the microsurgical procedure. The provision of the adjustable robotic manipulator arms and the monitoring unit via the control system allows for personalized configurations to accommodate individual preferences.

The monitoring unit 106 is configured, via the second control unit 114, to track a position of a surgical instrument carried by the at least one manipulator arm 108.

Advantageously, the second control unit 114 is configured to zoom in or zoom out the monitoring unit 106 depending on the movements and position of the at least one manipulator arm 108.

In particular, the second control unit 114 comprises an image processing unit.

The image processing unit advantageously includes advanced image processing techniques providing for enhanced visual representation of anatomical structures.

In particular, the second control unit 114 further comprises a display and/or a computing device.

The captured images or videos from the monitoring unit 106 (e.g. a digital microscope) are displayed on the display, e.g. a digital microscope screen.

Alternatively, or additionally, the captured images are displayed on an external computer connected to the control system 102.

For example, the monitoring unit 106 comprises a digital microscope that uses a camera and a digital imaging sensor to capture the images from the surgical area, which is then displayed on a dedicated digital microscope screen.

The second control unit 114 advantageously comprises a computing device including machine learning algorithms and the like.

In particular, machine learning algorithms can help enhance the precision and accuracy of robotic surgical systems. By analyzing large amounts of data, these algorithms can learn patterns and optimize surgical procedures, leading to improved surgical outcomes and reduced errors.

Advantageously, the use of the machine learning technology in combination with vision and motion control mechanisms significantly improves overall efficiency of the surgical robotic system. In particular, a safe and real-time guidance is facilitated during surgery, helping surgeons navigate complex anatomical structures and avoid critical areas.

The control system 102 further comprises a computing device for controlling the monitoring unit. For example, the computing device is included in the second control unit 114.

The computing device is configured to adjust the properties of the monitoring unit 106, such as a focus point, an autofocus and/or a zooming range.

The control system 102 comprises a controller that is, for example, included in the second control unit 114 configured to control the movement of the robotic assembly.

In this way, the real-time positioning of the monitoring unit can be precisely controlled. For example, the surgeon is enabled to move the monitoring unit to a desired position allowing better viewing of the surgical area.

For example, the computing device is configured for controlling the monitoring unit, for adjusting a focus point, an autofocus and/or a zooming range.

The surgical robotic system 100 further comprises a storage unit for storing the captured images and/or videos and/or position data of the surgical instruments. The storage unit, for example, is included in the second control unit 114.

The recording capabilities allow surgeons to capture images or videos of the microsurgical procedure. These recordings serve as valuable documentation for future reference, teaching, research or machine learning purposes.

In particular, the surgical robotic system 100 or the first control unit 112 comprises a motion control unit. The motion control unit is configured to control movements and positioning of the at least one manipulator arm 106.

In particular, the interface unit 116 comprises the connection between the surgical robotic system 100 and the monitoring unit 106. This includes the power connection, signal input and output lines, structural components for mounting the monitoring unit 106 to the surgical robotic system 100.

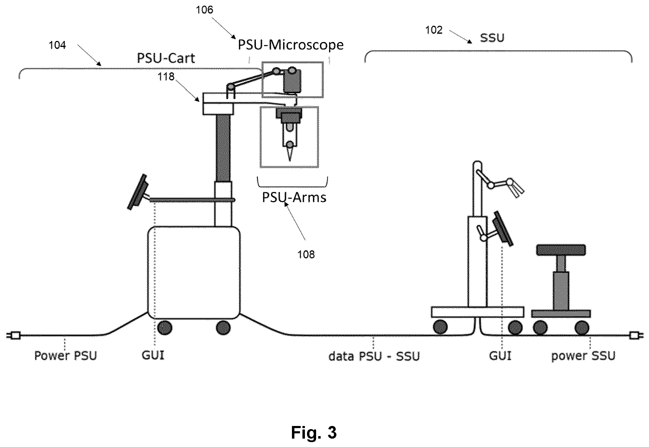

**Fig. 3** shows a schematic view of the surgical robotic system 100, indicating the control system 102 and the robotic system 104.

The robotic system 104 (e.g. PSU-cart) comprises the monitoring unit 106 (e.g. PSU-microscope) and the at least one manipulator arm 108 (e.g. PSU-arm) that are coupled to the support frame 118.

The control system 102 (e.g. SSU) of the surgical robotic system 100 is configured for controlling the robotic system 104, in particular the monitoring unit 116 and the at least one manipulator arm 108.

The control system 102 and the robotic system 104 may further comprise (are equipped with) a user interface 126 (e.g. a graphical user interface) and a power unit.

The control system and the robotic system are configured to be displaced (i.e. are designed to be movable), allowing optimal positioning.

Fig. 4 illustrates a schematic view of the robotic system 100, indicating a portion of the robotic system 104 including the at least one manipulator arm 108 and the monitoring unit 116.

For example, the robotic system 104 comprises two manipulator arms 108 that have a common reference frame with the monitoring unit 106.

In particular, the manipulator arm 108 is mounted onto a support frame 118 (e.g. a suspension frame).

Each of the manipulator arms 108 comprises a first arm 120 having a first end that is connected to an end effector 128. The end effector 128 is configured to hold a surgical instrument.

The manipulator arm 108 further comprises a second arm 122 that is connected to a second end of the first arm 120.

The first arm 120 is connected to the end effector 128 via a first joint and to the second arm 122 via a second joint.

In particular, the monitoring unit 106 is configured to be mounted onto the support frame 118.In particular, the monitoring unit 106 is removably mounted onto a mounting surface provided on the support frame 118.

For example, the monitoring unit 106 is mounted onto a centre of the mounting surface.

Advantageously, the monitoring unit 106 and the at least one manipulator arm 108 have the same frame of reference. This, in turn, improves the positioning accuracy relative to the patient and the treatment area.

It is further envisaged to employ additional markers or sensors disposed on the surgical instruments and the patient to further improve accuracy and safety of the microsurgical procedures.

For example, the monitoring unit 106 is mounted onto the support frame 118 via the robotic assembly 110.

The manipulator arm 108 further comprises a third arm 124 that is connected to the second arm 122 via a third joint.

In particular, the first, second and third joints, each comprise a roll and a pitch.

Optionally, the monitoring unit 106 is configured to be mounted onto the manipulators arm, for example, on the first, second or third arms as appropriate.

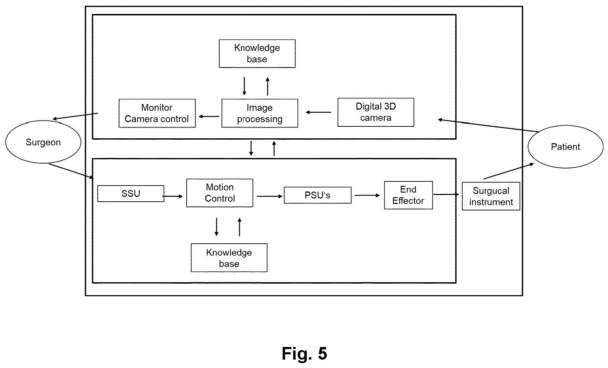

**Fig. 5** illustrates a functional block diagram of the surgical robotic system 100.

In the surgical robotic system 100 according to the present invention, the functionalities of the surgical robotic system and the real-time visualisation system (i.e. the monitoring system 106) are combined together. This design provides for one single interface unit

Surgeons are provided with a central control system which enables them to perform / have accurate control of the microsurgical procedures through the manipulator arm and the monitoring unit. Also, here, the motion control can be assisted by machine learning algorithms. Therefore, the motion control unit of the visualization system 100 may also comprise a knowledge base, e.g. a data storage and data processing system with e.g. motion control data or motion control related data, which will be used inter alia to enhance the motion control.

The surgeon interacts with the system through monitors displaying a 3D feed of the surgical site and control interfaces that allow them to guide the surgery with precision.

The SSU translates the surgeon's actions into machine commands, which are sent to the motion control unit.

The motion control unit receives visual data from the digital camera (optionally or additionally from another digital microscope), displaying high-resolution images on the monitor for the surgeon's reference.

The motion control adjusts the robotic system accordingly, ensuring the end effector (the robotic arm and instruments) performs the precise movements necessary for the surgery.

The visual information can be also sent to an image processing unit for further refinement and analysis.

For example, by way of the image processing the captured images are enhanced, thereby providing clearer visuals and additional insights that aid the surgeon in making informed decisions during surgery.

The processing, for example, can also integrate data from the knowledge base, which serves as a central repository of surgical protocols, best practices, and real-time analysis algorithms. The knowledge base optimizes the system's performance by offering data-driven support and adjustments during the procedure.

The knowledge base, for example, can incorporate real-time data analysis capabilities, utilizing algorithms to process and interpret incoming information from cameras and sensors. Machine learning models that can be incorporated within the knowledge base further enhances its functionality, providing predictive insights based on prior surgeries and helping to refine the precision of the robot's movements.

The provision of one central control system for controlling the monitoring unit and processing the image data, and for controlling the surgical instruments carried by the manipulator arm not only improves the real-time visualization of the surgical area, but also enables them to perform the surgical procedures at a higher precision level.

In other words, the visualization system provides critical real-time visual feedback from the targeted surgical area, enabling surgeons to perform intricate procedures with enhanced precision and accuracy. The combination of advanced imaging, visualization, and user-friendly interaction interface further contributes to the success and efficacy of the surgical robotic system according to the present invention.

The visualization system 106 may further incorporate image processing algorithms and machine learning algorithms to enhance the quality and analysis of the captured visuals. These algorithms can further be adapted to reduce noise and optimize contrast and color balance. Therefore, the image processing unit of the visualization system 106 may also comprise a knowledge base, e.g. a data storage and data processing system with e.g. image data or image related data, which will be used inter alia to enhance the visualization.

For example, image enhancement techniques can be implemented to help surgeons clearly visualize important details, such as blood vessels, nerves, or tissue boundaries.

Advantageously, the surgical instruments 130 that are held by the manipulator arm and are tracked by the monitoring unit are designed to mimic the movements of the surgeon's hand, but with enhanced dexterity and stability.

The integration of the microscope unit (e.g. including camera and control unit) into the microsurgical robotic system addresses the challenges faced by surgeons when conducting delicate microsurgical procedures and enhances their capabilities by providing real-time visual feedback and by improving depth perception.

Establishing a common frame of reference for the microscope and the robotic manipulator arm can help the surgeons to significantly improve their vision and the precision of their surgical maneuvers. The monitoring unit (e.g. the microscope) can provide accurate and reliable real-time spatial information of the surgical area, thereby enabling surgeons to have a better vision allowing for more precise manipulation of delicate tissues with the surgical instruments.

### REFERENCE NUMERALS

- 100: Surgical robotic system
- 102: Control system, SSU
- 104: Robotic system, PSU
- 106: Monitoring unit
- 108: Manipulator arm
- 110: Robotic assembly
- 112: First control unit
- 114: Second control unit
- 116: Interface unit
- 118: Support frame
- 120: First arm
- 122: Second arm
- 124: Third arm
- 126: User interface
- 128: End effector
- 130: Surgical Instrument

## Claims

1. A surgical robotic system (100) for performing a microsurgery or supermicrosurgery, comprising:
a control system (102) and a robotic system (104),
wherein the robotic system (104) comprises:
- a monitoring unit (106) for monitoring a surgical area, and
- at least one manipulator arm (108) for manipulating a surgical instrument, wherein the control system (102) is configured to control the monitoring unit (106) and the at least one manipulator arm (108), and
wherein the monitoring unit (106) and the at least one manipulator arm (108) are configured and designed to have a common reference frame.

2. The surgical robotic system (100) according to claim 1,
**characterized in that**
the monitoring unit (106) and the at least one manipulator arm (108) are configured to be mounted onto a support frame (118), preferably the monitoring unit (106) is configured to be mounted onto the manipulator arm (108) via a robotic assembly (110).

3. The surgical robotic system according to claims 1 or 2,
**characterized in that**
the control system (102) comprises:
- a first control unit (112) for controlling movements of the at least one manipulator arm (108),
- a second control unit (114) for positioning and controlling the monitoring unit (106), and
- an interface unit (116) in communication with the first control unit (112) and the second control unit (114).

4. The surgical robotic system (100) according to any one of the preceding claims,
**characterized in that**
the monitoring unit (106), via the control system (102) or the second control unit (114), is configured to track a position of a surgical instrument carried by the at least one manipulator arm (108), and/or to zoom in or zoom out depending on the movements of the at least one manipulator arm (108).

5. The surgical robotic system (100) according to any one of the preceding claims,
**characterized in that**
the at least one manipulator arm (108) comprises
- a first arm (120) having a first end that is connected to an end effector (128) and
- a second end that is connected to a second arm (122),
wherein the end effector (128) is configured to hold the surgical instrument.

6. The surgical robotic system (100) according to claim 5,
**characterized in that**
the support frame (118) include a mounting surface, wherein the monitoring unit (118) is mounted onto a centre of the mounting surface.

7. The surgical robotic system (100) according to claims 5 or 6,
**characterized in that**
the first arm (120) is connected to the end effector (128) via a first joint and to the second arm (122) via a second joint.

8. The surgical robotic system (100) according to claims 5, 6 or 7,
**characterized in that**
the at least one manipulator arm (108) further comprises a third arm (124) that is connected to the second arm via a third joint, preferably the first, second and third joints, each comprising a roll and a pitch.

9. The surgical robotic system (100) according to any one of the preceding claims,
**characterized in that**
the control system (102) or the second control unit (114) comprises a computing device for controlling the monitoring unit, wherein the computing device is configured to adjust a focus point, an autofocus and/or a zooming range.

10. The surgical robotic system (100) according any one of the preceding claims,
**characterized in that**
the control system (102) or the second control unit (114) further comprises a controller for controlling movements of the robotic assembly.

11. The surgical robotic system (100) according to any one of the preceding claims,
**characterized in that**
the control system (102) or the second control unit (114) further comprises at least one of an image processing unit, a display, a storage unit, and a knowledge base.

12. The surgical robotic system (100) according to any one of the preceding claims,
**characterized in that**
the control system (102) or the first control unit (112) comprises a motion control unit for controlling movements of the at least one manipulator arm (108).

13. The surgical robotic system (100) according to any one of the preceding claims,
**characterized in that**
the monitoring unit (106) comprises a digital imaging sensor and/or a digital 3D camera.
